(19) Europäisches Patentamt / European Patent Office / Office européen des brevets

(11) **EP 2 949 347 B1**

(12) **EUROPEAN PATENT SPECIFICATION**

(45) Date of publication and mention
of the grant of the patent:
**16.06.2021 Bulletin 2021/24**

(51) Int Cl.:
*A61L 27/56* (2006.01)　　*A61L 27/58* (2006.01)
*A61L 27/60* (2006.01)　　*A61F 2/10* (2006.01)

(21) Application number: **14742948.4**

(22) Date of filing: **21.01.2014**

(86) International application number:
**PCT/JP2014/051139**

(87) International publication number:
**WO 2014/115732 (31.07.2014 Gazette 2014/31)**

(54) **ARTIFICIAL DERMIS FOR TRANSPLANTATION AND METHOD FOR PRODUCING SAME**

KÜNSTLICHE DERMIS FÜR TRANSPLANTATION UND VERFAHREN ZUR HERSTELLUNG DAVON

DERME ARTIFICIEL POUR TRANSPLANTATION ET PROCÉDÉ POUR PRODUIRE CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.01.2013 JP 2013012626**

(43) Date of publication of application:
**02.12.2015 Bulletin 2015/49**

(73) Proprietors:
• **FUJIFILM Corporation**
**Tokyo 106-8620 (JP)**
• **Japan Tissue Engineering Co., Ltd.**
**Gamagori-shi, Aichi 443-0022 (JP)**

(72) Inventors:
• **OGIWARA, Kazutaka**
**Ashigarakami-gun**
**Kanagawa 258-8577 (JP)**
• **YAMAGUCHI, Kazuhiro**
**Ashigarakami-gun**
**Kanagawa 258-8577 (JP)**
• **SASAKI, Tasuku**
**Ashigarakami-gun**
**Kanagawa 258-8577 (JP)**
• **GOTO, Eiko**
**Gamagori-shi**
**Aichi 443-0022 (JP)**
• **SUGAWARA, Katsura**
**Gamagori-shi**
**Aichi 443-0022 (JP)**
• **HATA, Ken-ichiro**
**Gamagori-shi**
**Aichi 443-0022 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 923 078**　　**EP-A1- 2 159 259**
**WO-A1-2008/103043**　　**JP-A- H05 176 983**
**JP-A- 2010 053 080**

• **DATABASE WPI Week 200064 Thomson
Scientific, London, GB; AN 2000-659170
XP002770403, & JP 2000 262610 A (TERUMO
CORP) 26 September 2000 (2000-09-26)**
• **NAOKI MORIMOTO ET AL: "Novel
Collagen/Gelatin Scaffold with Sustained
Release of Basic Fibroblast Growth Factor:
Clinical Trial for Chronic Skin Ulcers", TISSUE
ENGINEERING PART A, vol. 19, no. 17-18, 1
September 2013 (2013-09-01), pages 1931-1940,
XP055374930, US ISSN: 1937-3341, DOI:
10.1089/ten.tea.2012.0634**
• **LIU H. ET AL.: 'Construction of chitosan-gelatin
-hyaluronic acid artificial skin in vitro.' JOURNAL
OF BIOMATERIALS APPLICATIONS vol. 21, no.
4, 2007, pages 413 - 430, XP008177458**
• **TSENG H.J. ET AL.: 'Characterization of
chitosan-gelatin scaffolds for dermal tissue
engineering.' JOURNAL OF TISSUE
ENGINEERING AND REGENERATIVE MEDICINE
vol. 7, no. 1, 28 October 2011, pages 20 - 31,
XP055211700 ISSN: 1932-6254 Retrieved from the
Internet: <URL:http://onlinelibrary.
wiley .com/doi/ 10.1002/term.492/full> [retrieved
on 2013-00-00]**

• WANG T.W. ET AL.: 'Biomimetic bilayered gelatin- chondroitin 6 sulfate-hyaluronic acid biopolymer as a scaffold for skin equivalent tissue engineering.' ARTIFICIAL ORGANS vol. 30, no. 3, 2006, pages 141 - 149, XP055211702

Remarks:

The file contains technical information submitted after the application was filed and not included in this specification

SEQUENCE LISTING

<110> FUJIFILM CORPORATION

<110> JAPAN TISSUE ENGINEERING CO., LTD

<120> Artificial Dermis and Method of Producing the Same

<130> FS-F06048-00

<150> JP 2013-012626
<151> 2013-01-25

<160> 1

<170> PatentIn version 3.5

<210> 1
<211> 571
<212> PRT
<213> Artificial

<220>
<223> CBE3

<400> 1

```
Gly Ala Pro Gly Ala Pro Gly Leu Gln Gly Ala Pro Gly Leu Gln Gly
1               5               10              15

Met Pro Gly Glu Arg Gly Ala Ala Gly Leu Pro Gly Pro Lys Gly Glu
            20              25              30

Arg Gly Asp Ala Gly Pro Lys Gly Ala Asp Gly Ala Pro Gly Ala Pro
            35              40              45

Gly Leu Gln Gly Met Pro Gly Glu Arg Gly Ala Ala Gly Leu Pro Gly
        50              55              60

Pro Lys Gly Glu Arg Gly Asp Ala Gly Pro Lys Gly Ala Asp Gly Ala
65              70              75              80

Pro Gly Lys Asp Gly Val Arg Gly Leu Ala Gly Pro Ile Gly Pro Pro
                85              90              95

Gly Glu Arg Gly Ala Ala Gly Leu Pro Gly Pro Lys Gly Glu Arg Gly
            100             105             110

Asp Ala Gly Pro Lys Gly Ala Asp Gly Ala Pro Gly Lys Asp Gly Val
            115             120             125

Arg Gly Leu Ala Gly Pro Ile Gly Pro Pro Gly Pro Ala Gly Ala Pro
    130             135             140
```

Gly Ala Pro Gly Leu Gln Gly Met Pro Gly Glu Arg Gly Ala Ala Gly
145                 150                 155                 160

Leu Pro Gly Pro Lys Gly Glu Arg Gly Asp Ala Gly Pro Lys Gly Ala
                165                 170                 175

Asp Gly Ala Pro Gly Lys Asp Gly Val Arg Gly Leu Ala Gly Pro Pro
                180                 185                 190

Gly Ala Pro Gly Leu Gln Gly Ala Pro Gly Leu Gln Gly Met Pro Gly
                195                 200                 205

Glu Arg Gly Ala Ala Gly Leu Pro Gly Pro Lys Gly Glu Arg Gly Asp
                210                 215                 220

Ala Gly Pro Lys Gly Ala Asp Gly Ala Pro Gly Ala Pro Gly Leu Gln
225                 230                 235                 240

Gly Met Pro Gly Glu Arg Gly Ala Ala Gly Leu Pro Gly Pro Lys Gly
                245                 250                 255

Glu Arg Gly Asp Ala Gly Pro Lys Gly Ala Asp Gly Ala Pro Gly Lys
                260                 265                 270

Asp Gly Val Arg Gly Leu Ala Gly Pro Ile Gly Pro Pro Gly Glu Arg
                275                 280                 285

Gly Ala Ala Gly Leu Pro Gly Pro Lys Gly Glu Arg Gly Asp Ala Gly
                290                 295                 300

Pro Lys Gly Ala Asp Gly Ala Pro Gly Lys Asp Gly Val Arg Gly Leu
305                 310                 315                 320

Ala Gly Pro Ile Gly Pro Pro Gly Pro Ala Gly Ala Pro Gly Ala Pro
                325                 330                 335

Gly Leu Gln Gly Met Pro Gly Glu Arg Gly Ala Ala Gly Leu Pro Gly
                340                 345                 350

Pro Lys Gly Glu Arg Gly Asp Ala Gly Pro Lys Gly Ala Asp Gly Ala
                355                 360                 365

Pro Gly Lys Asp Gly Val Arg Gly Leu Ala Gly Pro Pro Gly Ala Pro
                370                 375                 380

Gly Leu Gln Gly Ala Pro Gly Leu Gln Gly Met Pro Gly Glu Arg Gly
385                 390                 395                 400

Ala Ala Gly Leu Pro Gly Pro Lys Gly Glu Arg Gly Asp Ala Gly Pro
405 410 415

Lys Gly Ala Asp Gly Ala Pro Gly Ala Pro Gly Leu Gln Gly Met Pro
420 425 430

Gly Glu Arg Gly Ala Ala Gly Leu Pro Gly Pro Lys Gly Glu Arg Gly
435 440 445

Asp Ala Gly Pro Lys Gly Ala Asp Gly Ala Pro Gly Lys Asp Gly Val
450 455 460

Arg Gly Leu Ala Gly Pro Ile Gly Pro Pro Gly Glu Arg Gly Ala Ala
465 470 475 480

Gly Leu Pro Gly Pro Lys Gly Glu Arg Gly Asp Ala Gly Pro Lys Gly
485 490 495

Ala Asp Gly Ala Pro Gly Lys Asp Gly Val Arg Gly Leu Ala Gly Pro
500 505 510

Ile Gly Pro Pro Gly Pro Ala Gly Ala Pro Gly Ala Pro Gly Leu Gln
515 520 525

Gly Met Pro Gly Glu Arg Gly Ala Ala Gly Leu Pro Gly Pro Lys Gly
530 535 540

Glu Arg Gly Asp Ala Gly Pro Lys Gly Ala Asp Gly Ala Pro Gly Lys
545 550 555 560

Asp Gly Val Arg Gly Leu Ala Gly Pro Pro Gly
565 570

**Description**

Technical Field

The invention relates to artificial dermis for transplantation and a method for producing the same.

Background Art

Refractory skin ulcers are caused by diabetes, arterial insufficiency, venous insufficiency, decubitus, trauma, or the like. In particular, along with the recent increase in the number of diabetic patients, diabetic skin ulcers, one of the complications of diabetes, are steadily increasing. It is difficult to cure refractory skin ulcers by a treatment such as a conservative treatment, and therefore major, invasive surgery such as flap transplantation or quadruple amputation is often required.

As one of the treatments, a treatment employing artificial dermis consisting primarily of a biodegradable material such as collagen is currently provided. The artificial dermis acts as scaffolds in dermis regeneration. As a result, a dermis structure (dermis-like granulation tissue) is formed by invasion and proliferation of fibroblasts and blood vessels from a graft bed, whereby a pseudo-dermis of a patient is regenerated, while the biodegradable material constituting the artificial dermis is degraded to be absorbed.

Making porous a wound contact layer consisting of a biodegradable material of artificial dermis having a porous structure can promote substitution of the layer by one's own tissue. Artificial skin in which a moisture permeation controlling layer and the like are layered on such a wound contact layer is known (for example, see Japanese Patent Application Laid-Open (JP-A) Nos. H05-184662 and H05-285211).

SUMMARY OF INVENTION

Technical Problem

Artificial dermis comprising gelatin are known, see Tseng et al (J Tissue Eng Regen Med 2013; 7: 20-31) and JP-A No. H05-176983. Artificial dermis comprising recombinant gelatin is also known, see EP2159259 A1. The conventional artificial skin having a porous structure as described in JP-A No. H05-184662 or H05-285211 has an ability to form a dermis-like tissue (granulation-like tissue) at a defective part of skin. However, artificial dermis having greater ability to form a dermis-like tissue (granulation tissue) is desired.

The invention addresses provision of artificial dermis for transplantation that has an excellent ability to form a dermis-like tissue (granulation-like tissue) at a defective part of skin.

The invention also addresses provision of a method for producing the artificial dermis for transplantation.

Solution to Problem

As a result of intensive studies of artificial dermis by the present inventors to solve the above problem, we found that the artificial dermis exhibits an excellent ability to form a dermis-like tissue (granulation-like tissue) when a wound contact layer including a porous sheet consisting of a biodegradable material has a specific porous structure, and completed the invention.

The artificial dermis for transplantation according to the invention has a wound contact layer including a porous sheet consisting of a biodegradable material, in which the porous sheet satisfies the following Equation:

$$\text{Equation: } X2/X1 \leq 0.8.$$

In the porous sheet, when a line is drawn parallel to a wound contact face and a line of equal length is drawn perpendicular to the wound contact face, X1 represents the number of points of intersection between the line parallel to the wound contact face and pore walls; and X2 represents the number of points of intersection between the line perpendicular to the wound contact face and pore walls, wherein the X2/X1 is from 0.1 to 0.8 and wherein the biodegradable material is a recombinant gelatin.

The method for producing artificial dermis for transplantation according to the invention includes a step of freezing an aqueous solution of a biodegradable material in the production of the porous sheet.

Advantageous Effects of Invention

The artificial dermis according to the invention includes a porous sheet having a specific porous structure, thereby exhibiting an excellent ability to form a dermis-like tissue (granulation-like tissue) at a defective part of skin, due to adopting a specific porous structure of a porous sheet. Furthermore, the method for producing artificial dermis for transplantation according to the invention enables simple production of the artificial dermis according to the invention using a known production means.

BRIEF DESCRIPTION OF DRAWING

Fig. 1 is an optical micrograph showing an internal, cross-sectional structure of a porous sheet that was produced by a recombinant gelatin and stained with eosin in Example 1.
Fig. 2 is an optical micrograph (enlarged view: 1,500 $\mu$m $\times$ 1,500 $\mu$m) showing an internal, cross-sectional structure of a porous sheet that was produced by a recombinant gelatin and stained with eosin in Example 1.
Fig. 3 is a histological image with HE staining obtained in a dermis-like tissue (granulation-like tissue) formation test using a porous sheet produced by a recombinant gelatin in Example 1.
Fig. 4 is an optical micrograph showing an internal, cross-sectional structure of a porous sheet that was produced by a recombinant gelatin and stained with eosin in Example 2.
Fig. 5 is an optical micrograph (enlarged view: 1,500 $\mu$m $\times$ 1,500 $\mu$m) showing an internal, cross-sectional structure of a porous sheet produced by a recombinant gelatin and stained with eosin in Example 2.
Fig. 6 is a histological image with HE staining obtained in a dermis-like tissue (granulation-like tissue) formation test using the porous sheet produced by a recombinant gelatin in Example 2.
Fig. 7 is an optical micrograph showing an internal, cross-sectional structure of a porous sheet that was produced by a recombinant gelatin and stained with eosin in Comparative Example 1.
Fig. 8 is an optical micrograph (enlarged view: 1,500 $\mu$m $\times$ 1,500 $\mu$m) showing an internal, cross-sectional structure of the porous sheet that was produced by a recombinant gelatin and stained with eosin in Comparative Example 1.
Fig. 9 is a histological image with HE staining obtained in a dermis-like tissue (granulation-like tissue) formation test using a porous sheet produced by a recombinant gelatin in Comparative Example 1.
Fig. 10 is an optical micrograph showing an internal, cross-sectional structure of a porous sheet of Comparative Example 2 that was stained with eosin.
Fig. 11 is an optical micrograph (enlarged view: 1,500 $\mu$m $\times$ 1,500 $\mu$m) showing an internal, cross-sectional structure of a porous sheet of Comparative Example 2 that was stained with eosin.
Fig. 12 is a histological image with HE staining obtained in a dermis-like tissue (granulation-like tissue) formation test using a porous sheet of Comparative Example 2.

DESCRIPTION OF EMBODIMENTS

Hereinbelow, embodiments of the invention are described in detail. Here, the following explanation and examples merely illustrate the invention and are not intended to limit the scope of the invention.
In this specification, a numerical range described as "(from) ... to ..." represents a range including the numerical values before and after "to" as the minimum value and the maximum value, respectively.
As used herein, the term "step" indicates not only a separate step but also a step that is not clearly distinguished from another step or steps as long as the desired effect of the step is obtained therefrom.
In this specification, an amino acid residue in an amino acid sequence is sometimes represented by a single-letter amino acid code (for example, a glycine residue is represented by "G") or a three-letter amino acid code (for example, a glycine residue is represented by "Gly") known in the art.
In the invention, "%" with regard to an amino acid sequence of a protein or a polypeptide is based on the number of amino acid residues unless otherwise specified.
In this specification, "homology" with regard to amino acid sequences may be a value calculated using a BLAST package (see Ausubel et al. 1999, Short Protocols in Molecular Biology, 4th Ed. Chapter 18). For example, 80% homology or more with the amino acid sequence represented by SEQ ID NO: 1 means that a value of Max. Identities in BLAST is 80 or more.
The artificial dermis for transplantation according to the invention (hereinafter, sometimes referred to as "artificial dermis according to the invention") has a wound contact layer that includes a porous sheet consisting of a biodegradable material, in which the porous sheet satisfies the following Equation:

Equation: $X2/X1 \leq 0.8$.

In the porous sheet, when a line is drawn parallel to a wound contact face and a line of equal length is drawn perpendicular to the wound contact face, X1 represents a number of points of intersection between the line parallel to the wound contact face and pore walls, and X2 represents a number of points of intersection between the line perpendicular to the wound contact face and pore walls, wherein the X2/X1 is from 0.1 to 0.8 and wherein the biodegradable material is a recombinant gelatin.

In order to determine X1 and X2, it is preferable to draw three parallel lines and three perpendicular lines, and it is preferable that each of the lines has a length of 1,500 $\mu$m and that the total length of the three parallel lines is 4,500 $\mu$m and the total length of the three perpendicular lines is 4,500 $\mu$m. That is, in the porous sheet, in a case in which three lines (three lines each having a length of 1,500 $\mu$m; total length of 4,500 $\mu$m) parallel to the wound contact face and three lines (three lines each having a length of 1,500 $\mu$m; total length of 4,500 $\mu$m) perpendicular to the wound contact face are drawn, X1 represents a number of points of intersection between the three lines parallel to the wound contact face and pore walls, and X2 represents a number of points of intersection between the three lines perpendicular to the wound contact face and pore walls.

For example, X1 and X2 can be determined by acquiring a cross-sectional image of a 1,500 $\mu$m $\times$ 1,500 $\mu$m square built upon a line parallel to the wound contact face and a line perpendicular to the wound contact face, and drawing three parallel lines and three perpendicular lines that equally divide the cross-sectional image into 16 squares. That is, in a case in which a cross-sectional image (a square of 1,500 $\mu$m $\times$ 1,500 $\mu$m) built upon a line parallel to the wound contact face and a line perpendicular to the wound contact face is acquired from the porous sheet, and three parallel lines and three perpendicular lines equally dividing the cross-sectional image into 16 squares are drawn, X1 represents a number of points of intersection between the three lines parallel to the wound contact face and pore walls, and X2 represents a number of points of intersection between the three lines perpendicular to the wound contact face and pore walls. It is preferable to acquire the cross-sectional image of a square of 1,500 $\mu$m $\times$ 1,500 $\mu$m from the central region of the porous sheet.

More specifically, X1 and X2 are determined as follows.

In a case in which the wound contact face of the porous sheet is substantially flat, a plane parallel to the flat face (hereinafter, referred to as "parallel plane") and a plane perpendicular to the flat face (hereinafter, referred to as "perpendicular plane") are arbitrarily selected, and a cross-sectional image of a 1,500 $\mu$m $\times$ 1,500 $\mu$m square whose two sides are respectively on the parallel plane and the perpendicular plane is acquired. With respect to each of the side formed on the parallel plane and the side formed on the perpendicular plane, among the sides of the acquired cross-sectional image of the square, three parting lines perpendicular thereto are drawn so that each of the sides are equally divided into quarters. Here, a parting line dividing the side formed on the parallel plane is referred to as "perpendicular line", and a parting line dividing the side formed on the perpendicular plane is referred to as "parallel line". A perpendicular line is a line perpendicular to the wound contact face, and a parallel line is a line parallel to the wound contact face. In the cross-sectional image, X1 represents the total number of points of intersection between the three parallel lines and pore-forming wall portions, and X2 represents the total number of points of intersection between the three perpendicular lines and pore-forming wall portions.

In a case in which the wound contact face of the porous sheet is not substantially flat, the wound contact face is deformed to be flat. Thereafter, the artificial dermis is placed on a flat surface with the wound contact face facing downward, and a plane parallel to the flat face (parallel plane) and a plane perpendicular to the flat face (perpendicular plane) are arbitrarily selected, and a cross-sectional image of a 1,500 $\mu$m $\times$ 1,500 $\mu$m square whose two sides are on the parallel plane and the perpendicular plane is acquired. X1 and X2 are determined based on the cross-sectional image of the 1,500 $\mu$m $\times$ 1,500 $\mu$m square by the method described above.

In a case in which the wound contact face of the porous sheet is not substantially flat and it is impossible to deform the wound contact face to be flat, the artificial dermis is placed on a flat surface with the wound contact face facing downward, and then a plane parallel to the flat face (parallel plane) and a plane perpendicular to the flat face (perpendicular plane) are arbitrarily selected, and a cross-sectional image of a 1,500 $\mu$m $\times$ 1,500 $\mu$m square whose two sides are on the parallel plane and the perpendicular plane is acquired. X1 and X2 are determined based on the cross-sectional image of the 1,500 $\mu$m $\times$ 1,500 $\mu$m square by the method described above.

X2/X1 is a parameter representing the pore shape of the porous sheet. A smaller value of X2/X1, the value being less than 1, means that the number of pore-forming wall portions is relatively small in a thickness direction, and means, for example, that the shape of each of the pores is elongated in a thickness direction. Meanwhile, a value of X2/X1 that is closer to 1 means that pore-forming wall portions in a thickness direction and pore-forming wall portions in a parallel direction are evenly distributed, and means, for example, that the shape of each of the pores is isotropic. A larger value of X2/X1, the value being more than 1, means that the number of pore-forming wall portions is relatively small in a planar direction, and means, for example, that the shape of each of the pores is elongated in a planar direction.

The artificial dermis according to the invention is characterized in that X2/X1 is 0.8 or less and that the number of pore-forming wall portions is relatively small in a thickness direction. As a result, fibroblasts and blood vessels invade and proliferate more easily in a graft bed, and an excellent ability, such as an ability to form a dermis-like tissue (granulation-like tissue) in a short time, can be exhibited. Due to the excellent ability to form a dermis-like tissue (granulation-like tissue), a dermis-like tissue (granulation-like tissue) can be formed even when no growth factor and/or cell are used. However, embodiments of the artificial dermis of the invention do not exclude an aspect in which a growth factor and/or a cell are included.

In the invention, X2/X1 is 0.1 to 0.8. The range of the upper limit of X2/X1 is preferably 0.7 or less, more preferably 0.6 or less, still more preferably 0.5 or less, and even more preferably 0.4 or less. The range of the lower limit of X2/X1 is 0.1 or more, more preferably 0.2 or more, and still more preferably 0.3 or more. The range of X2/X1 is preferably from 0.1 to 0.8, more preferably from 0.2 to 0.7, still more preferably from 0.3 to 0.6, and even more preferably from 0.3 to 0.4.

An upper limit within the above range is preferable since a greater ability to form a dermis-like tissue (granulation-like tissue), which is an effect of the invention, can be obtained. A lower limit within the above range is preferable from the viewpoint of the physical strength of the artificial dermis for transplantation.

The value of X1 (the total number of points of intersection between the three parallel lines each having a length of 1,500 $\mu$m and pore-forming wall portions) is not particularly limited, and preferably from 10 to 500, more preferably from 30 to 300, still more preferably from 40 to 150, and even more preferably from 50 to 100.

The value of X2 (the total number of points of intersection between the three perpendicular lines each having a length of 1,500 $\mu$m and pore-forming wall portions) is not particularly limited, and the preferable range thereof is determined based on the preferable range for X2/X1 and the preferable range of X1.

*Biodegradable Material*

The type of the biodegradable material according to the invention is degradable *in vivo*. With regard to the biodegradable material used in the invention, hydropathicity represented by the Grand average of hydropathicity (GRAVY) value is preferably from 0.0 to 5.0, and more preferably from 0.3 to 3.0. In particular, a hydrophilic polypeptide that satisfies the above range is preferable. The GRAVY value can be obtained by a method described in "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571-607" or "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788 (2003)".

The biodegradable material is recombinant gelatin.

*Recombinant Gelatin*

A recombinant gelatin is highly biocompatible, which is a natural property of a gelatin, and has an excellent property of being non-infectious, since a recombinant gelatin is a non-natural compound and therefore has low risk of infection such as BSE (Bovine Spongiform Encephalopathy). Since a recombinant gelatin has more uniform quality than a naturally occurring gelatin, and the sequence of a recombinant gelatin can be determined, a recombinant gelatin can be designed precisely in terms of strength and degradability without large variations by crosslinking or the like described below.

The molecular weight of the recombinant gelatin is preferably from 2 kDa to 100 kDa, more preferably from 2.5 kDa to 95 kDa, still more preferably from 5 kDa to 90 kDa, and even more preferably from 10 kDa to 90 kDa.

The recombinant gelatin preferably contains a repeat of a sequence represented by Gly-X-Y (GXY sequence) characteristic to collagen. Here, Gly represents a glycine residue; each of X and Y independently represents an arbitrary amino acid residue (preferably an arbitrary amino acid residue other than a glycine residue); and a plurality of sets of Gly-X-Y may be the same as or different from one another. The GXY sequences characteristic to collagen are a highly specific partial structure found in the amino acid composition and sequence of gelatin and collagen, that is virtually absent in other proteins. Glycine residues account for approximately one-third of the partial structure, and the partial structure has an amino acid sequence in which glycine repeatedly appears every three amino acid residues. Glycine is an amino acid having the simplest structure. There are few restrictions in the arrangement of glycine in the molecular chain, and glycine highly contributes to regeneration of the helix structure upon gelatinization. It is preferable that imino acid residues (e.g., a proline residue, an oxyproline residue) account for a substantial portion of the amino acid residues represented by X or Y, and that imino acid residues account for 10% to 45% of the whole recombinant gelatin. The repeated structure of the GXY sequence accounts for preferably 80% or more, more preferably 95% or more, and still more preferably 99% or more of the amino acid residues of the whole recombinant gelatin.

Generally available gelatins contain charged polar amino acids and uncharged polar amino acids at a ratio of 1:1. Here, a polar amino acid specifically refers to cysteine, aspartic acid, glutamic acid, histidine, lysine, asparagine, glutamine, serine, threonine, tyrosine, or arginine. Among these, uncharged polar amino acids refer to cysteine, asparagine, glutamine, serine, threonine, and tyrosine. In the recombinant gelatin used in the invention, the percentage of polar amino acid residues with respect to the total amino acid residues constituting the recombinant gelatin is preferably from 10% to 40%, and more preferably from 20% to 30%. Meanwhile, the percentage of uncharged polar amino acid residues with respect to polar amino acid residues is preferably from 5% to less than 20% and more preferably from 7% to less than 10%. It is preferable that the amino acid sequence does not contain one, preferably two or more amino acid residues, selected from serine, threonine, asparagine, tyrosine, or cysteine.

In general, a minimal amino acid sequence that functions as a cell adhesion signal sequence in a protein or a polypeptide is known. It is preferable that the recombinant gelatin used in the invention has, in the molecule thereof, at least two minimal amino acid sequences that function as cell adhesion signal sequences. More specifically, the minimal amino acid sequence represented by the single-letter amino acid code is preferably an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, or an HAV sequence, more preferably an RGD sequence, a YIGSR sequence, a PDSGR sequence, an LGTIPG sequence, an IKVAV sequence, or an HAV sequence, and still more preferably an RGD sequence, since various kinds of cells can adhere to the above sequences. Among RGD sequences, an ERGD sequence is preferable.

In the recombinant gelatin used in the invention, the number of occurrences of the minimal amino acid sequence in a single molecule of a protein is preferably from 3 to 50, more preferably from 4 to 30, still more preferably from 5 to 20, and even more preferably 12, in view of adhesion property and proliferative ability of the cells.

In a case in which the recombinant gelatin used in the invention contains occurrences of RGD sequence (sometimes referred to as "RGD motif'), with regard to the arrangement of the RGD sequences, the number of amino acid residues present between two RGD sequences is preferably from 0 to 100 and more preferably from 25 to 60. It is preferable that the number of amino acid residues present between two RGD sequences is uneven.

In the recombinant gelatin used in the invention, the percentage of the RGD motif portions with respect to the total number of the amino acid residues (total number of amino acid residues in the RGD motif portions/the total number of the amino acid residues $\times$ 100) is preferably at least 1.2%. In a case in which the recombinant gelatin contains 250 or more amino acid residues, it is preferable that each stretch of 250 amino acid residues contains at least one RGD motif. A percentage of the RGD motif of 1.2% corresponds to occurrences of at least one RGD sequence per 250 amino acid residues. For example, in order to meet the percentage of at least 1.2%, a gelatin consisting of 251 amino acid residues is required to contain at least two RGD sequences.

The percentage of the RGD motif with respect to the total number of the amino acid residues is more preferably at least 1.8%, still more preferably at least 2.4%, even more preferably at least 3.0%, yet more preferably at least 3.6%, and most preferably at least 4.5%. The number of the RGD motif in the recombinant gelatin per 250 amino acid residues is preferably at least 2, more preferably at least 3, still more preferably at least 4, even more preferably at least 6, yet more preferably at least 8, and further more preferably from 12 to 16.

The recombinant gelatin used in the invention may be partially hydrolyzed.

It is preferable that the recombinant gelatin used in the invention has an amino acid sequence of A-[(Gly-X-Y)$_n$]$_m$-B. Here, A represents one, two or more arbitrary amino acid residues; B represents one, two or more arbitrary amino acid residues; Gly represents a glycine residue; X, n in number, each independently represents an arbitrary amino acid residue; Y, n in number, each independently represents an arbitrary amino acid residue; n represents an integer from 3 to 100; and m represents an integer from 2 to 10. With regard to n sets of Gly-X-Y, all of them may be the same as one another, or some but not all of them may be the same as one another, or each of them may be different from one another. n is preferably from 15 to 70, and more preferably from 50 to 65. m is preferably from 3 to 5.

As the one or more arbitrary amino acid residues represented by A in the amino acid sequence, a glycine residue or 2 to 6 amino acid residues containing at least one glycine residue are preferable, and 3 amino acid residues containing at least one glycine residue is more preferable.

As the one or more arbitrary amino acid residues represented by B in the amino acid sequence, a glycine residue or 2 to 6 amino acid residues containing at least one glycine residue are preferable, and a glycine residue is more preferable.

With regard to a repeat unit contained in the recombinant gelatin, the repeat unit in which a plurality of naturally occurring collagen sequence units are linked is preferable. Here, the naturally occurring collagen may be any naturally occurring collagen, and preferable examples thereof include type-1, type-II, type-III, type-IV, and type-V collagens. More preferable examples thereof include type-I, type-II, and type-III collagens. In another embodiment, the origin of the collagen is preferably a human, a bovine, a pig, a mouse, or a rat, and more preferably a human.

The isoelectric point of the recombinant gelatin used in the invention is preferably from 5 to 10, more preferably from 6 to 10, and still more preferably from 7 to 9.5.

It is preferable that the recombinant gelatin is not deaminated.

It is preferable that the recombinant gelatin has no telopeptide.

It is preferable that the recombinant gelatin is a substantially pure collagen-like material prepared using a nucleic acid encoding a naturally occurring collagen.

The recombinant gelatin used in the invention preferably has the following amino acid sequence.

Gly-Ala-Pro-[(Gly-X-Y)$_{63}$]$_3$-Gly

Gly represents a glycine residue; Ala represents an alanine residue; Pro represents a proline residue; each of the 63 occurrences of X independently represents an arbitrary amino acid residue; and each of the 63 occurrences of Y independently represents an arbitrary amino acid residue. With regard to 63 sets of Gly-X-Y, all of them may be the same as one another, or some but not all of them may be the same as one another, or each of them may be different from one another.

The recombinant gelatin used in the invention is more preferably a recombinant gelatin indicated by the following (1) or (2).

(1) a recombinant gelatin having the amino acid sequence represented by SEQ ID NO: 1.

(2) a recombinant gelatin which has an amino acid sequence having 80% or more (more preferably 90% or more, and still more preferably 95% or more) homology with the amino acid sequence represented by SEQ ID NO: 1, and which is biodegradable.

SEQ ID NO: 1:

GAP(GAPGLQGAPGLQGMPGERGAAGLPGPKGERGDAGPKGADGAPGAPGLQGMP

GERGAAGLPGPKGERGDAGPKGADGAPGKDGVRGLAGPIGPPGERGAAGLPGPKGE

RGDAGPKGADGAPGKDGVRGLAGPIGPPGPAGAPGAPGLQGMPGERGAAGLPGPKG

ERGDAGPKGADGAPGKDGVRGLAGPP)$_3$G

The recombinant gelatin used in the invention can be produced by a gene recombination technique known to those skilled in the art, and, for example, can be produced according to the method described in EP1014176A2, US6992172B1, WO2004/85473A2, or WO2008/103041A1. More specifically, a gene encoding an amino acid sequence of a predetermined recombinant gelatin is obtained, the gene is incorporated into an expression vector to prepare a recombinant expression vector, and then the vector is introduced into an appropriate host, thereby obtaining a transformant. The obtained transformant is cultured in an appropriate medium to produce a recombinant gelatin. Therefore, the recombinant gelatin used in the invention can be prepared by collecting the produced recombinant gelatin from the culture.

*Cross-linking*

The biodegradable material in the invention may be a cross-linked material or a non-cross-linked material, and is preferably a cross-linked material. The cross-linking method may be a known method, such as thermal cross-linking, chemical cross-linking, cross-linking using an aldehyde (e.g., formaldehyde, glutaraldehyde), cross-linking using a condensing agent (e.g., carbodiimide, cyanamide), enzymatic cross-linking, photocrosslinking, or UV cross-linking. In addition to the cross-linking (covalent binding), it is also preferable that the recombinant gelatin is formed in a higher order structure due to at least one of hydrophobic interaction, hydrogen bond, or ionic interaction.

*Porous Sheet*

Hereinbelow, the porous sheet consisting of a biodegradable material in the invention is described.

*(a) Porosity*

Porosity (P (%)) is obtained by Equation: P = 1 - p/pc (%), based on a bulk density (p) and a true density (pc). The bulk density (p) can be calculated from its dry weight and volume. The true density (pc) can be determined by a specific gravity bottle method using a Hubbard-type specific gravity bottle. The porosity in the invention is preferably from 81% to 99.99%, and more preferably from 95.01% to 99.9%.

*(b) Hole Interconnecting Pores*

A hole interconnecting pores may be present in the porous sheet according to the invention. The presence of the hole interconnecting pores allows pores to communicate with each other from the surface of the porous sheet through the deep portion of the porous sheet, as a result of which fibroblasts and blood vessels invade and proliferate more easily in a graft bed.

*(c) Water Absorption Rate*

Water absorption rate (%) can be calculated from Equation: $W1/W0 \times 100$ (%), based on a dry weight (W0) and a weight at the time of water swelling (W1) measured after 5-minute spontaneous absorption of ultrapure water at 25°C and sufficient removal of surplus water on a plastic petri dish. The water absorption rate is preferably from 1000% to 9900%, and more preferably from 2000% to 5000%.

The porous sheet consisting of a biodegradable material can be produced using a known method. For example, the porous sheet consisting of a biodegradable material can be produced by freezing a solution (preferably an aqueous solution) in which the biodegradable material is dissolved in a solvent, followed by freeze-drying and thermal cross-linking. The porosity, pore size, X1, X2, and X2/X1 of the porous sheet vary depending on the type of the biodegradable material to be used, conditions for freezing and freeze-drying (e.g., the concentration of the aqueous solution of the biodegradable material before freezing, the amount of the aqueous solution, freezing speed, freezing temperature), or the like. By adjusting them, an intended porosity, pore size, X1, X2, and X2/X1 can be obtained. For example, in a case in which the freezing speed in freezing is set to a faster speed, X2/X1 tends to be lower.

The porous sheet in the invention forms a wound contact layer. When the wound contact face of the porous sheet contacts with a wound, invasion and proliferation of fibroblasts and blood vessels occur in the porous sheet. Therefore, the porous sheet functions as artificial dermis that exhibits an excellent ability to form a dermis-like tissue (granulation-like tissue). For this reason, it is preferable that no other layer is formed on the wound contact face of the porous sheet. However, another layer or layers may be formed on the wound contact face of the porous sheet as long as the another layer or layers does not inhibit the ability to form a dermis-like tissue (granulation-like tissue) of the artificial dermis according to the invention.

In the wound contact layer according to the invention, another layer or layers may be formed on the face opposite to the wound contact face. Examples of the another layer or layers include a known layer that artificial dermis is provided with, and specific examples thereof include a vapor transmission controlling layer, a silicone layer, and a mesh layer.

The artificial dermis according to the invention may contain a growth factor and/or a cell or the like. For example, a growth factor and/or a cell or the like may be contained in the porous sheet (that is, in the wound contact layer). However, the artificial dermis according to the invention provides excellent ability to form a dermis-like tissue (granulation-like tissue) even when no growth factor and/or cell or the like is contained. Therefore, it is preferable to contain no growth factor and/or cell from the viewpoint of production cost and the like.

The artificial dermis according to the invention can be preferably used for a defective part of skin, a defective part of mucosa, or the like.

The method of producing artificial dermis for transplantation according to the invention is a method for producing the artificial dermis for transplantation according to the invention, including a step of freezing an aqueous solution of a biodegradable material (freezing step) in the production of the porous sheet. The method of producing the artificial dermis for transplantation according to the invention preferably further includes a step of freeze-drying the frozen aqueous solution of the biodegradable material (freeze-drying step), and more preferably further includes a step of cross-linking the biodegradable material after freeze-drying (cross-linking step). The cross-linking step is preferably a step of cross-linking the biodegradable material after freeze-drying by heating (thermal cross-linking step). The methods used for freezing and subsequent freeze-drying and cross-linking may be known methods.

In the method of producing artificial dermis for transplantation according to the invention, the porosity, pore size X1, X2, and X2/X1 of the porous sheet can be adjusted by controlling the type of the biodegradable material to be used, conditions for freezing and freeze-drying (e.g., the concentration of the aqueous solution of the biodegradable material before freezing, the amount of the aqueous solution, freezing speed, freezing temperature), or the like.

EXAMPLES

Hereinafter, the invention is described more specifically with reference to Examples, but the invention is not limited to these examples.

*Example 1*

*Recombinant Gelatin*

As a recombinant gelatin, CBE3 described below was prepared (CBE3 is described in WO2008/103041A1).

CBE3

- Molecular weight: 51.6 kD
- Structure: $GAP[(GXY)_{63}]_3G$
- The number of amino acids: 571
- The number of RGD sequences: 12
- Imino acid content: 33%
- The repeated structure of the GXY accounts for substantially 100% of the amino acids.
- The amino acid sequence of CBE3 contains none of serine, threonine, asparagine, tyrosine, or cysteine.
- CBE3 include an ERGD sequence.
- Isoelectric point: 9.34
- Amino acid sequence (SEQ ID NO: 1 in the Sequence Listing)

GAP(GAPGLQGAPGLQGMPGERGAAGLPGPKGERGDAGPKGADGAPGAPGLQGMP
GERGAAGLPGPKGERGDAGPKGADGAPGKDGVRGLAGPIGPPGERGAAGLPGPKGE
RGDAGPKGADGAPGKDGVRGLAGPIGPPGPAGAPGAPGLQGMPGERGAAGLPGPKG
ERGDAGPKGADGAPGKDGVRGLAGPP)$_3$G

*Production of Porous Sheet*

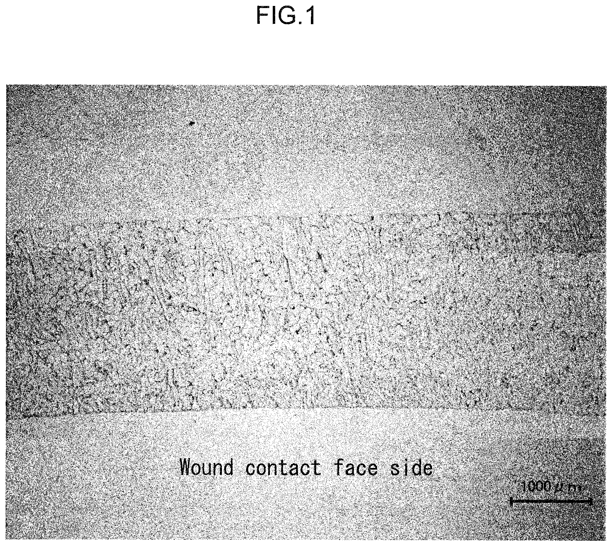

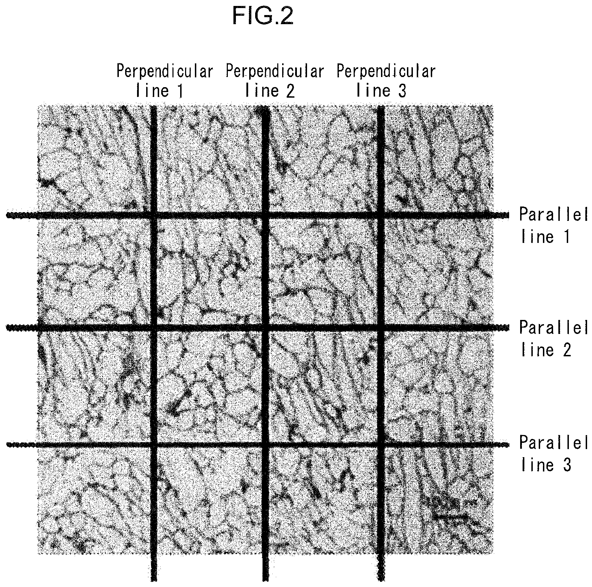

A 3% aqueous solution of gelatin was prepared using the recombinant gelatin CBE3, and poured into an aluminum cup (diameter: 53 mm × height: 37 mm). The solution was left to stand for 1 hour on a rack of an aluminum block provided with a glass plate thereon in a freezer at -80°C. After freeze-drying the resultant, thermal cross-linking was carried out at 160°C for 3 hours, thereby obtaining a porous sheet (average thickness: 2,300 μm) consisting of the recombinant gelatin. The obtained porous sheet was stained with eosin, and the structure of the resultant was confirmed under an optical microscope (see Fig. 1). Fig. 2 is an enlarged view of 1,500 μm × 1,500 μm, and three parallel lines and three perpendicular lines were drawn to determine respective numbers of points of intersection according to the above-described method. X1, which is the total number of points of intersection between the three parallel lines and pore-forming wall portions, was 98 (parallel line 1: the number of points of intersection was 37, parallel line 2: the number of points of intersection was 30, parallel line 3: the number of points of intersection was 31). X2, which is the total number of points of intersection between the three perpendicular lines and pore-forming wall portions, was 68 (perpendicular line 1: the number of points of intersection was 19, perpendicular line 2: the number of points of intersection was 25, perpendicular 3: the number of points of intersection was 24). X2/X1 was 0.7. In the present embodiment, the artificial dermis is composed only of a wound contact layer which is the porous sheet.

*Test for Dermis-like Tissue (Granulation-like Tissue) Formation on the Back of a Rat*

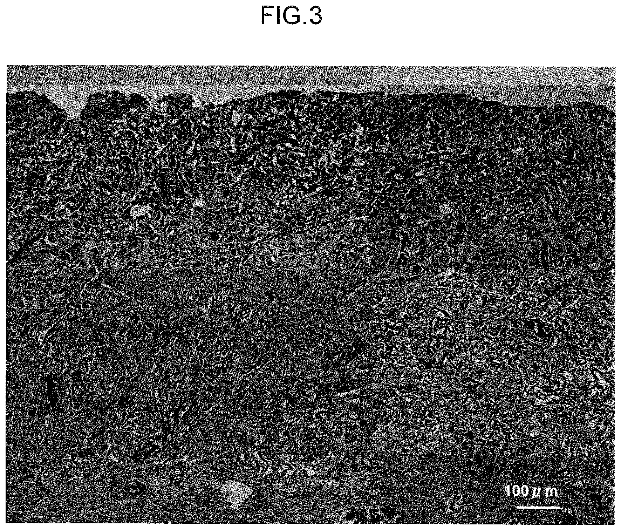

90 mg/kg of ketamine hydrochloride and 10 mg/kg of xylazine hydrochloride were administered intraperitoneally to anesthetize a Wistar rat (male, older than 10 week-old). The hair on the back of the rat was removed, and a circular defective part of the dermis with a diameter of 19 mm was created. The obtained artificial dermis was applied to the defective part of the dermis, and then Vaseline gauze (registered trade name: ADAPTIC, Johnson & Johnson), wet cotton, and gauze were layered in this order and fixed. In the second week, the back tissue including the artificial dermis was isolated under anesthesia. As a result of histological observation of the isolated tissue by HE (Hematoxylin Eosin) staining, dermis-like tissue (granulation-like tissue) formation was observed significantly compared to those observed in Comparative Examples 1 and 2 described below (see Fig. 3).

*Example 2*

*Production of Porous Sheet*

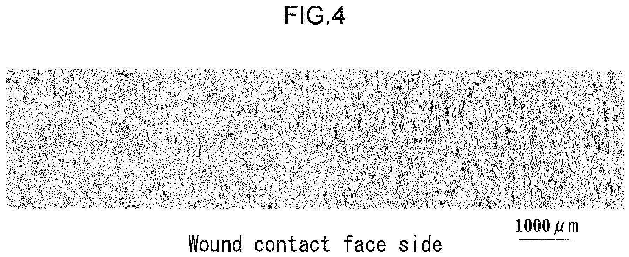

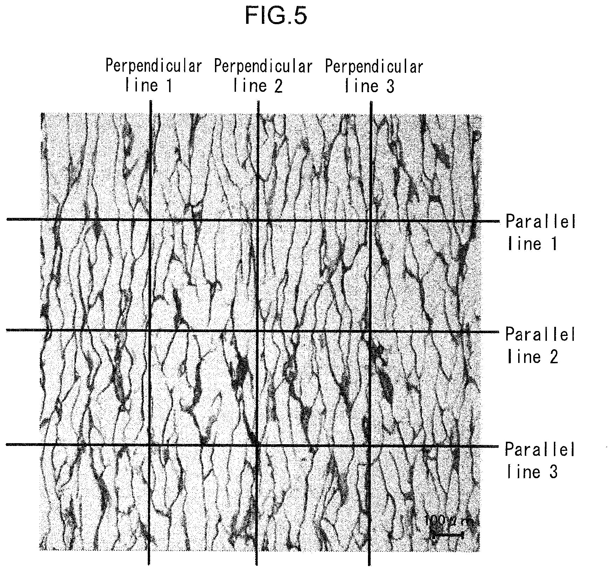

A 3% aqueous solution of gelatin was prepared using the recombinant gelatin CBE3, and poured into an aluminum cup (diameter: 53 mm × height: 37 mm). The solution was left to stand for 1 hour on a rack of an aluminum block provided with a glass plate thereon in a freezer at -60°C. After freeze-drying the resultant, thermal cross-linking was carried out at 160°C for 3 hours, thereby obtaining a porous sheet (average thickness: 2,500 μm) consisting of the recombinant gelatin. The obtained porous sheet was stained with eosin, and the structure of the resultant was confirmed under an optical microscope (see Fig. 4). Fig. 5 is an enlarged view of 1,500 μm × 1,500 μm, and three parallel lines and three perpendicular lines were drawn to determine respective numbers of points of intersection according to the above-described method. X1, which is the total number of points of intersection between the three parallel lines and pore-forming wall portions, was 108 (parallel line 1: number of points of intersection: 36, parallel line 2: number of points of intersection: 34, parallel line 3: number of points of intersection: 38). X2, which is the total number of points of intersection between the three perpendicular lines and pore-forming wall portions, was 32 (perpendicular line 1: number of points of intersection: 11, perpendicular line 2: number of points of intersection: 7, perpendicular 3: number of points of intersection: 14). X2/X1 was 0.3. In the present embodiment, the artificial dermis is composed only of the wound contact layer which is the porous sheet.

*Test for Dermis-like Tissue (Granulation-like Tissue) Formation on the Back of a Rat*

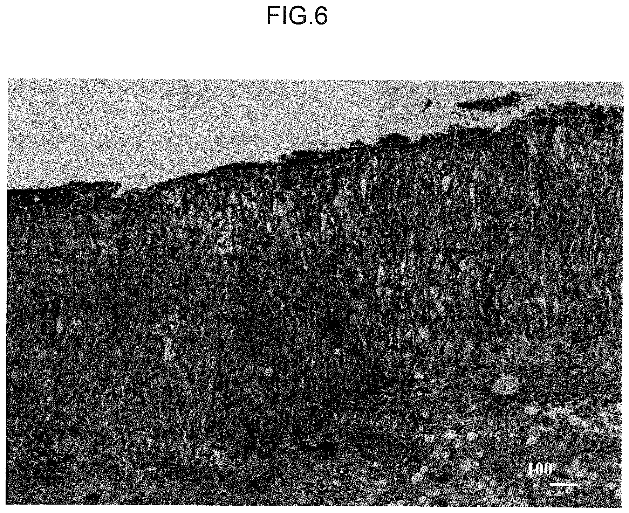

Evaluation was carried out in a manner similar to Example 1 (see Fig. 6). Dermis-like tissue (granulation-like tissue) formation was observed significantly compared to those observed in Comparative Examples 1 and 2 described below. Furthermore, dermis-like tissue (granulation-like tissue) formation equal to or greater than that in Example 1 was observed.

*Comparative Example 1*

*Production of Porous Sheet*

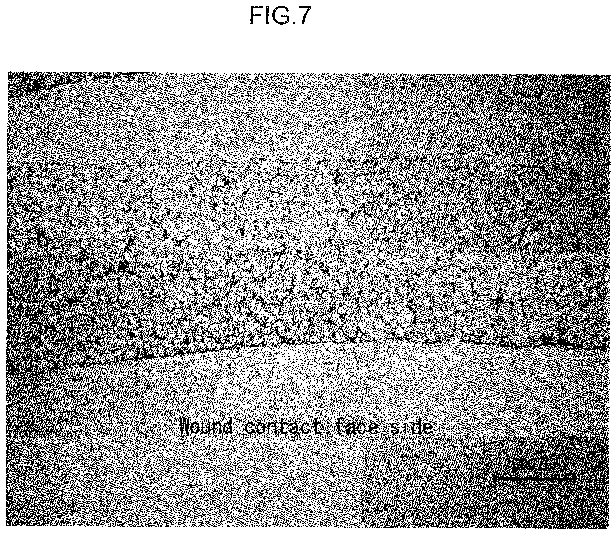

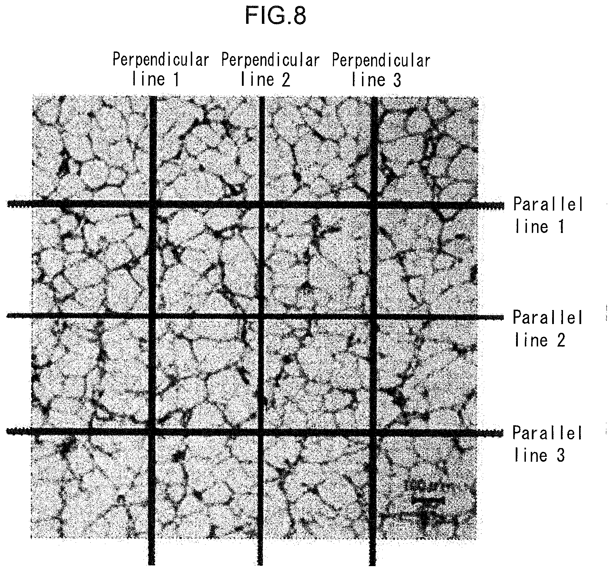

A porous sheet (average thickness: 2,200 μm) consisting of the recombinant gelatin was obtained in a manner similar to Example 1, except that "in a freezer at -80°C" in Example 1 was changed to "in a freezer at -40°C". The obtained porous sheet was stained with eosin, and the structure of the resultant was observed under an optical microscope (see Fig. 7). Fig. 8 is an enlarged view of 1,500 μm × 1,500 μm, and three parallel lines and three perpendicular lines were drawn to determine respective numbers of points of intersection according to the above-described method. XI, which is the total number of points of intersection between the three parallel lines and pore-forming wall portions, was 58 (parallel line 1: number of points of intersection: 19, parallel line 2: number of points of intersection: 18, parallel line 3: number of points of intersection: 21). X2, which is the total number of points of intersection between the three perpendicular lines and a wall portion that forms a pore, was 57 (perpendicular line 1: number of points of intersection: 20, perpendicular line 2: number of points of intersection: 19, perpendicular 3: number of points of intersection: 18). X2/X1 was 1. In the present embodiment, the artificial dermis is composed only of the wound contact layer which is the porous sheet.

*Test for Dermis-like Tissue (Granulation-like Tissue) Formtion on the Back of a Rat*

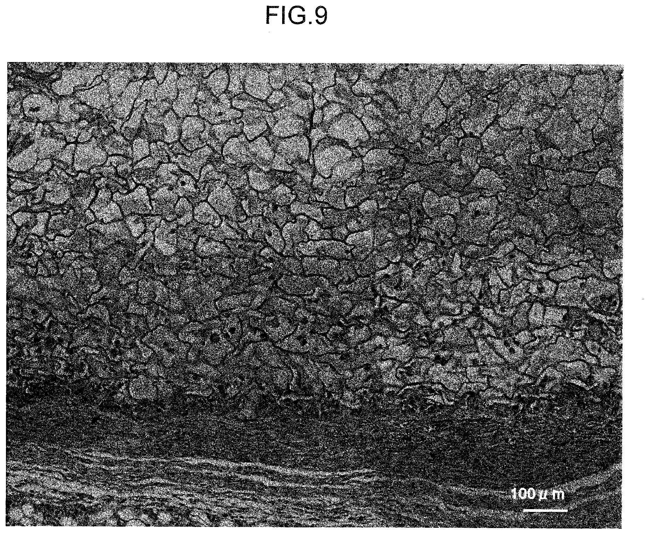

Evaluation was carried out in a manner similar to Example 1 (see Fig. 9). It was found that dermis-like tissue (granulation-like tissue) formation was more noticeable in Examples 1 and 2.

*Comparative Example 2*

*Production of Porous Sheet*

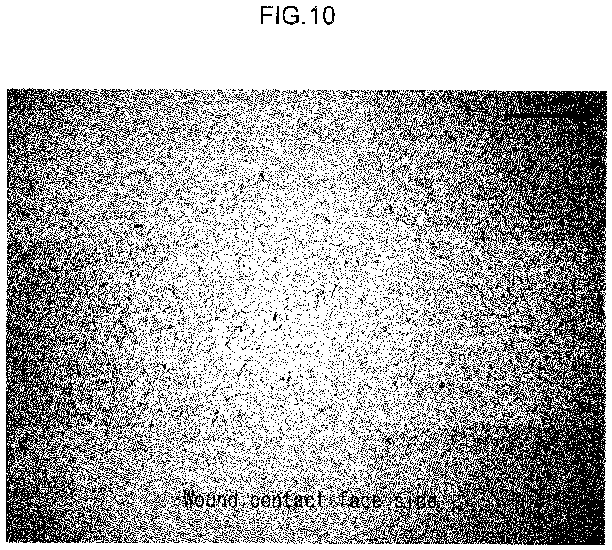

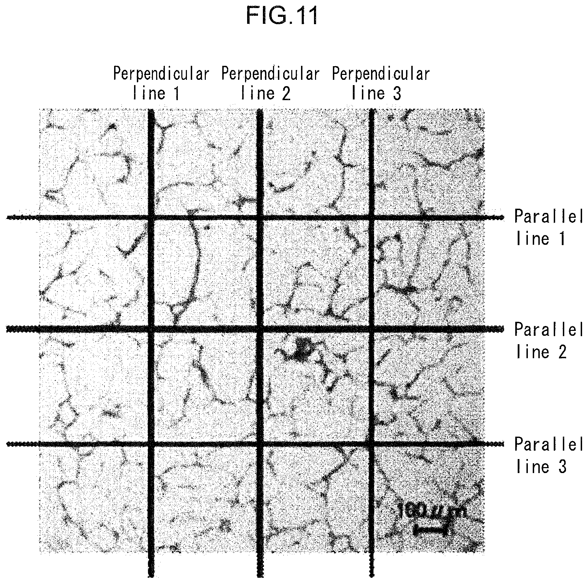

Test was carried out in a manner similar to Example 1, except that a commercially available graft for dermal defect, TERUDERMIS (registered trade name, manufactured by Olympus Terumo Biomaterials Corp.) (average thickness: 3,300 μm), was used instead of the artificial dermis of Example 1. The TERUDERMIS was stained with eosin, and the structure thereof was confirmed under an optical microscope (see Fig. 10). Fig. 11 is an enlarged view of 1,500 μm × 1,500 μm, and three parallel lines and three perpendicular lines were drawn to determine respective numbers of points of intersection according to the above-described method. X1, which is the total number of points of intersection between the three parallel lines and a wall portion that forms a pore, was 41 (parallel line 1: number of points of intersection:

13, parallel line 2: number of points of intersection: 12, parallel line 3: number of points of intersection: 16). X2, which is the total number of points of intersection between the three perpendicular lines and a wall portion that forms a pore, was 40 (perpendicular line 1: number of points of intersection: 12, perpendicular line 2: number of points of intersection: 13, perpendicular 3: number of points of intersection: 15). X2/X1 was 1. In the present embodiment, the artificial dermis is composed only of the wound contact layer which is the porous sheet.

*Test for Dermis-like Tissue (Granulation-like Tissue) Formation on the Back of a Rat*

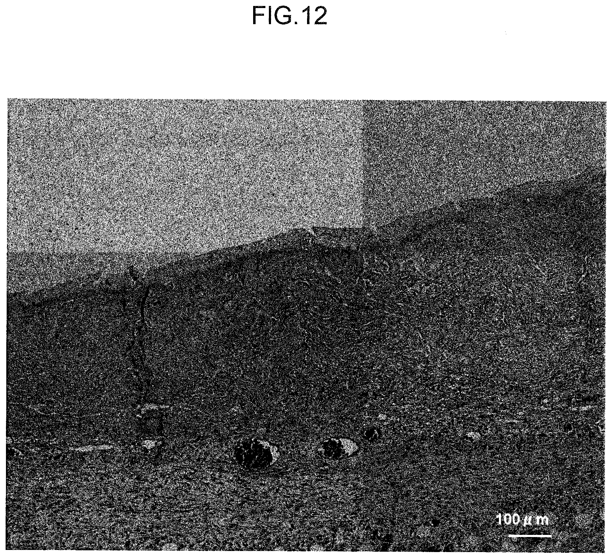

Evaluation was carried out in the same manner as that in Example 1 (see Fig. 12). It was found that dermis-like tissue (granulation-like tissue) formation was more noticeable in Examples 1 and 2.

As shown in the above, the artificial dermis according to Examples of the invention exhibits an excellent ability to form the dermis-like tissue (granulation-like tissue) by having a specific porous structure.

### Claims

1. Artificial dermis for transplantation, comprising a wound contact layer that includes a porous sheet consisting of a biodegradable material, the porous sheet satisfying the following Equation:

$$\text{Equation: } X2/X1 \leq 0.8,$$

wherein, in the porous sheet, when a line is drawn parallel to a wound contact face and a line of equal length is drawn perpendicular to the wound contact face, X1 represents a number of points of intersection between the line parallel to the wound contact face and pore walls, and X2 represents a number of points of intersection between the line perpendicular to the wound contact face and pore walls, wherein the X2/X1 is from 0.1 to 0.8 wherein the biodegradable material is a recombinant gelatin.

2. The artificial dermis for transplantation according to claim 1, wherein the recombinant gelatin has the following amino acid sequence:

A-[(Gly-X-Y)$_n$]$_m$-B,

wherein, A represents one or more arbitrary amino acid residues; B represents one or more arbitrary amino acid residues; X, n in number, each independently represents an arbitrary amino acid residue; Y, n in number, each independently represents an arbitrary amino acid residue; n represents an integer from 3 to 100; m represents an integer from 2 to 10; and all of the n sets of Gly-X-Y may be the same as one another, or some but not all of the n sets of Gly-X-Y may be the same as one another, or each of the n sets of Gly-X-Y may be different from one another.

3. The artificial dermis for transplantation according to claim 1 or claim 2, wherein the recombinant gelatin has the following amino acid sequence:

Gly-Ala-Pro-[(Gly-X-Y)$_{63}$]$_3$-Gly,

wherein, each of the 63 occurrences of X independently represents an arbitrary amino acid residue; each of the 63 occurrences of Y independently represents an arbitrary amino acid residue; and all 63 sets of Gly-X-Y may be the same as one another, or some but not all of the 63 sets of Gly-X-Y may be the same as one another, or each of the 63 sets of Gly-X-Y may be different from one another.

4. The artificial dermis for transplantation according to any one of claims 1 to 3, wherein the recombinant gelatin is:

(1) a recombinant gelatin having an amino acid sequence represented by SEQ ID NO: 1, or
(2) a recombinant gelatin that has an amino acid sequence having 80% or more homology with an amino acid sequence represented by SEQ ID NO: 1, and being biodegradable.

5. The artificial dermis for transplantation according to any one of claims 1 to 4, wherein the biodegradable material is cross-linked.

6. A method for producing the artificial dermis for transplantation according to any one of claims 1 to 5, the method including a step of freezing an aqueous solution of the biodegradable material in the production of the porous sheet.


**Patentansprüche**

1. Künstliche Dermis zur Transplantation, umfassend eine Wundkontaktschicht, die eine poröse Schicht (sheet) einschließt, die aus einem biologisch abbaubaren Material besteht, wobei die poröse Schicht die folgende Gleichung erfüllt:

Gleichung: X2/X1 $\leq$ 0,8,
wobei in der porösen Schicht, wenn eine Linie parallel zu einer Wundkontaktfläche gezogen wird und eine Linie gleicher Länge senkrecht zu der Wundkontaktfläche gezogen wird, X1 eine Anzahl von Schnittpunkten zwischen der Linie parallel zu der Wundkontaktfläche und den Porenwänden repräsentiert und X2 eine Anzahl von Schnittpunkten zwischen der Linie senkrecht zu der Wundkontaktfläche und den Porenwänden repräsentiert, wobei X2/X1 von 0,1 bis 0,8 beträgt
wobei das biologisch abbaubare Material eine rekombinante Gelatine ist.

2. Künstliche Dermis zur Transplantation gemäß Anspruch 1, wobei die rekombinante Gelatine die folgende Aminosäuresequenz aufweist:

A-[(Gly-X-Y)$_n$]$_m$-B,
wobei A einen oder mehrere beliebige Aminosäurereste repräsentiert; B einen oder mehrere beliebige Aminosäurereste repräsentiert; X, n an der Zahl, jeweils unabhängig einen beliebigen Aminosäurerest repräsentiert; Y, n an der Zahl, jeweils unabhängig einen beliebigen Aminosäurerest repräsentiert; n eine ganze Zahl von 3 bis 100 repräsentiert; m eine ganze Zahl von 2 bis 10 repräsentiert; und alle der n Sätze von Gly-X-Y gleich sein können, oder einige, aber nicht alle der n Sätze von Gly-X-Y gleich sein können, oder jeder der n Sätze von Gly-X-Y unterschiedlich sein kann.

3. Künstliche Dermis zur Transplantation gemäß Anspruch 1 oder Anspruch 2, wobei die rekombinante Gelatine die folgende Aminosäuresequenz aufweist:

Gly-Ala-Pro-[(Gly-X-Y)$_{63}$]$_3$-Gly,
wobei jedes der 63 auftretenden X unabhängig einen beliebigen Aminosäurerest repräsentiert; jedes der 63 auftretenden Y unabhängig einen beliebigen Aminosäurerest repräsentiert; und alle 63 Sätze von Gly-X-Y gleich sein können, oder einige, aber nicht alle der 63 Sätze von Gly-X-Y gleich sein können, oder jeder der 63 Sätze von Gly-X-Y voneinander verschieden sein kann.

**4.** Künstliche Dermis zur Transplantation gemäß irgendeinem der Ansprüche 1 bis 3, wobei die rekombinante Gelatine ist:

(1) eine rekombinante Gelatine mit einer Aminosäuresequenz, repräsentiert durch SEQ ID NO: 1, oder
(2) eine rekombinante Gelatine, die eine Aminosäuresequenz mit einer Homologie von 80% oder mehr mit einer durch SEQ ID NO: 1 repräsentierten Aminosäuresequenz aufweist und biologisch abbaubar ist.

**5.** Künstliche Dermis zur Transplantation gemäß irgendeinem der Ansprüche 1 bis 4, wobei das biologisch abbaubare Material vernetzt ist.

**6.** Verfahren zur Herstellung der künstlichen Dermis zur Transplantation gemäß irgendeinem der Ansprüche 1 bis 5, wobei das Verfahren einen Schritt des Einfrierens einer wässrigen Lösung des biologisch abbaubaren Materials bei der Herstellung der porösen Schicht umfasst.

## Revendications

**1.** Derme artificiel pour transplantation, comprenant une couche de contact avec la plaie qui inclut une feuille poreuse constituée d'un matériau biodégradable, la feuille poreuse satisfaisant à l'équation suivante :

$$\text{Équation : } X2/X1 \leq 0{,}8,$$

dans lequel, dans la feuille poreuse, lorsqu'une ligne est tracée parallèlement à une face de contact avec la plaie et qu'une ligne de longueur égale est tracée perpendiculairement à la face de contact avec la plaie, X1 représente un nombre de points d'intersection entre la ligne parallèle à la face de contact avec la plaie et les parois des pores, et X2 représente un nombre de points d'intersection entre la ligne perpendiculaire à la face de contact avec la plaie et les parois des pores, dans lequel le rapport X2/X1 est de 0,1 à 0,8, dans lequel le matériau biodégradable est une gélatine recombinante.

**2.** Derme artificiel pour transplantation selon la revendication 1, dans lequel la gélatine recombinante présente la séquence d'acides aminés suivante :
A-[(Gly-X-Y)$_n$]$_m$-B,
dans lequel, A représente un ou plusieurs résidus d'acide aminé arbitraires; B représente un ou plusieurs résidus d'acide aminé arbitraires ; X, en nombre n, chacun représente indépendamment un résidu d'acide aminé arbitraire ; Y, en nombre n, chacun représente indépendamment un résidu d'acide aminé arbitraire ; n représente un entier de 3 à 100 ; m représente un entier de 2 à 10 ; et tous les n ensembles de Gly-X-Y peuvent être identiques les uns aux autres, ou certains mais pas tous les n ensembles de Gly-X-Y peuvent être identiques les uns aux autres, ou chacun des n ensembles de Gly-X-Y peut être différent l'un de l'autre.

**3.** Derme artificiel pour transplantation selon la revendication 1 ou la revendication 2, dans lequel la gélatine recombinante présente la séquence d'acides aminés suivante :
Gly-Ala-Pro-[(Gly-X-Y)$_{63}$]$_3$-Gly,
dans lequel, chacune des 63 occurrences de X représente indépendamment un résidu d'acide aminé arbitraire ; chacune des 63 occurrences de Y représente indépendamment un résidu d'acide aminé arbitraire; et les 63 ensembles de Gly-X-Y peuvent être identiques les uns aux autres, ou certains mais pas tous les 63 ensembles de Gly-X-Y peuvent être identiques les uns aux autres, ou chacun des 63 ensembles de Gly-X-Y peut être différent l'un de l'autre.

**4.** Derme artificiel pour transplantation selon l'une quelconque des revendications 1 à 3, dans lequel la gélatine recombinante est :

(1) une gélatine recombinante présentant une séquence d'acides aminés représentée par SEQ ID NO : 1, ou
(2) une gélatine recombinante qui présente une séquence d'acides aminés présentant 80 % ou plus d'homologie avec une séquence d'acides aminés représentée par SEQ ID NO : 1, et étant biodégradable.

5. Derme artificiel pour transplantation selon l'une quelconque des revendications 1 à 4, dans lequel le matériau biodégradable est réticulé.

6. Procédé de production du derme artificiel pour transplantation selon l'une quelconque des revendications 1 à 5, le procédé incluant une étape de congélation d'une solution aqueuse du matériau biodégradable lors de la production de la feuille poreuse.

FIG.1

Wound contact face side

1000 μm

## FIG.2

FIG.3

FIG.4

Wound contact face side

$1000 \mu m$

## FIG.5

Perpendicular   Perpendicular   Perpendicular
line 1          line 2          line 3

Parallel line 1

Parallel line 2

Parallel line 3

FIG.6

# FIG.7

## FIG.8

Perpendicular line 1  Perpendicular line 2  Perpendicular line 3

Parallel line 1

Parallel line 2

Parallel line 3

FIG.9

FIG.10

Wound contact face side

FIG.11

FIG.12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H05184662 A
- JP H05285211 A
- JP H05176983 A
- EP 2159259 A1
- EP 1014176 A2
- US 6992172 B1
- WO 200485473 A2
- WO 2008103041 A1
- JP 2013012626 A

### Non-patent literature cited in the description

- **TSENG et al.** *J Tissue Eng Regen Med,* 2013, vol. 7, 20-31
- **AUSUBEL et al.** Short Protocols in Molecular Biology. 1999
- **GASTEIGER E. ; HOOGLAND C. ; GATTIKER A. ; DUVAUD S. ; WILKINS M.R. ; APPEL R.D. ; BAIROCH A.** Protein Identification and Analysis Tools on the ExPASy Server
- The Proteomics Protocols Handbook. Humana Press, 2005, 571-607
- ExPASy: the proteomics server for in-depth protein knowledge and analysis. **GASTEIGER E. ; GATTIKER A. ; HOOGLAND C. ; IVANYI I. ; APPEL R.D. ; BAIROCH A.** Nucleic Acids Res. 2003, vol. 31, 3784-3788